(19) **Europäisches Patentamt** / **European Patent Office** / **Office européen des brevets**

(11) **EP 4 450 609 A1**

(12) # EUROPEAN PATENT APPLICATION
published in accordance with Art. 153(4) EPC

(43) Date of publication:
**23.10.2024 Bulletin 2024/43**

(21) Application number: **22907476.0**

(22) Date of filing: **14.12.2022**

(51) International Patent Classification (IPC):
*C12M 3/00* *(2006.01)*    *C12N 5/10* *(2006.01)*
*C12N 1/00* *(2006.01)*    *C12P 21/08* *(2006.01)*
*C12M 1/00* *(2006.01)*    *C12M 1/107* *(2006.01)*
*C12M 1/21* *(2006.01)*

(52) Cooperative Patent Classification (CPC):
**C07K 16/00; C12M 1/00; C12M 1/107; C12M 1/21; C12M 3/00; C12N 1/00; C12N 5/10**

(86) International application number:
**PCT/JP2022/046000**

(87) International publication number:
**WO 2023/112952 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.12.2021 JP 2021203376**

(71) Applicant: **FUJIFILM Corporation**
**Tokyo 106-8620 (JP)**

(72) Inventors:
• **SAKUYAMA, Hiroshi**
**Ashigarakami-gun, Kanagawa 258-8577 (JP)**
• **TAKAHASHI, Naoto**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **METHOD FOR PRODUCING PRODUCT, AND CELL CULTURE APPARATUS**

(57) An object of the present invention is to provide a method for producing a product by cell culture, which makes it possible to suppress the clogging of an exhaust filter due to bubbles generated on an upper surface of a culture solution, and a cell culture device that makes it possible to suppress the clogging of an exhaust filter due to bubbles generated on an upper surface of a culture solution. According to the present invention, there is provided a method for producing a product, which includes culturing a cell using a cell culture device to produce a product from the cell, where the cell culture device includes a culture container accommodating a cell suspension containing cells and at least one or more bubble trap containers capable of accumulating bubbles, the one or more bubble trap containers being connected to an outside of the culture container.

FIG. 1

Processed by Luminess, 75001 PARIS (FR)

EP 4 450 609 A1

**Description**

Technical Field

The present invention relates to a method for producing a product including culturing a cell using a cell culture device. The present invention further relates to a cell culture device.

Background Art

In the production of a biopharmaceutical product, it is important to increase a cell density in a culture solution in order to improve the productivity of an antibody. In order to increase a cell density in a culture solution, it is necessary to dissolve a large amount of oxygen in the culture solution and supply a sufficient amount of oxygen to high-density cells. As a means for supplying a sufficient amount of oxygen to cells, a method of installing a sparger in a culture tank is known. In order to supply a sufficient amount of oxygen to cells, a general technique is such that oxygen is easily dissolved by making the pore diameter of the sparger as small as possible, and a large amount of oxygen is supplied to cells by supplying a large amount of oxygen. However, as the oxygen bubbles are smaller, the damage to the cells given by the energy generated in a case where the oxygen bubbles are ruptured is also increased. Therefore, it is difficult to supply oxygen to cells using microbubbles, and the fact is that, practically, a response is made by supplying a large amount of oxygen.

As a culture device in which air bubbles generated on a solution surface of a culture solution are difficult to flow into an exhaust port of the culture container, Patent Document 1 discloses a single-use culture device including a culture container in which a culture solution can be enclosed; an in-liquid aeration means that is disposed at a lower part of the culture container and carries out in-liquid aeration of oxygen to a culture solution enclosed in the culture container; an exhaust port that is positioned at an upper part of the culture container and discharges a gas in the culture container; and a shielding sheet that is disposed below the exhaust port and separates the exhaust port from a solution surface of the culture solution.

Prior Art Documents

Patent Documents

Patent Document 1: JP2018-19615A

**SUMMARY OF THE INVENTION**

**Object to be solved by the invention**

In a case where a large amount of oxygen is supplied from the sparger in order to supply a sufficient amount of oxygen to cells, a large quantity of bubbles is generated on the upper surface of the culture solution due to a surfactant component and cell-derived proteins which are contained in the culture solution. These bubbles finally fill the space on the upper surface of the culture solution and finally clog the exhaust filter associated with a bioreactor, which makes it impossible to continue the culture. The inventors of the present invention found that in the culture in which the cell density is increased, a problem to be solved is to control the bubbles generated on the upper surface of the culture solution by supplying a large amount of oxygen required by the cells.

An object to be achieved by the present invention is to provide a method for producing a product by cell culture, where the method makes it possible to suppress the clogging of an exhaust filter due to bubbles generated on an upper surface of a culture solution. Further, another object to be achieved by the present invention is to provide a cell culture device that makes it possible to suppress the clogging of an exhaust filter due to bubbles generated on an upper surface of a culture solution.

**Means for solving the object**

As a result of diligent studies to solve the above problem, the inventors of the present invention found that the above problems can be solved by installing a bubble trap container between a culture tank and an exhaust filter installed in an upper part of a culture tank, thereby completing the present invention.

That is, according to the present invention, the following inventions are provided.

<1> A method for producing a product, comprising:

culturing a cell using a cell culture device to produce a product from the cell,
in which the cell culture device includes a culture container accommodating a cell suspension containing cells and at least one or more bubble trap containers capable of accumulating bubbles, the one or more bubble trap containers being connected to an outside of the culture container.

<2> The method for producing a product according to <1>, in which at least one or more exhaust filters are connected to the bubble trap container.

<3> The method for producing a product according to <1> or <2>, in which a waste liquid line is connected to the bubble trap container.

<4> The method for producing a product according to any one of <1> to <3>, in which a discharge port for a gas which is aerated to the culture container is only a line that connects the culture container and the bubble trap container.

<5> The method for producing a product according to any one of <1> to <4>, in which the waste liquid line connected to the bubble trap container is connected to a waste liquid tank maintaining an aseptic state.

<6> The method for producing a product according to <5>, in which the waste liquid tank is allowed to be switched aseptically.

<7> The method for producing a product according to any one of <1> to <6>, in which an anti-foaming agent is added into the bubble trap container, or
a physical anti-foaming mechanism is provided in the bubble trap container.

<8> The method for producing a product according to any one of <1> to <7>, in which the following expression is satisfied,

$$\mathrm{Mi}^2 \times \mathrm{Ma}^{0.5}/\mathrm{S}/200 \leq \mathrm{Vt} \leq 10\,\mathrm{W}$$

in the expression,

Vt indicates a bubble trap container capacity (L),
Mi indicates an aeration amount (L/min) from a microsparger having a pore diameter of 1 $\mu$m to 200 $\mu$m,
Ma indicates an aeration amount (L/min) of a macrosparger having a pore diameter of 0.1 mm to 10 mm,
S indicates a gas-liquid interface area ($m^2$) of a culture solution, and
W indicates a culture solution amount (L) in the culture container.

<9> The method for producing a product according to any one of <1> to <8>, in which the following expression is satisfied,

$$\mathrm{L} \leq [\mathrm{P}^2/(\mathrm{Mi}^{1.5} + \mathrm{Ma})] \times \mathrm{S}^{0.5}$$

in the expression,

L indicates a length (m) of a tube that connects the bubble trap container and the culture container,
S indicates a gas-liquid interface area ($m^2$) of a culture solution,
P indicates a thickness (mm) of a tube that connects the bubble trap container and the culture container,
Mi indicates an aeration amount (L/min) from a microsparger having a pore diameter of 1 $\mu$m to 200 $\mu$m, and
Ma indicates an aeration amount (L/min) of a macrosparger having a pore diameter of 0.1 mm to 10 mm.

<10> The method for producing a product according to any one of <1> to <9>, in which the following expression is satisfied,

$$(\mathrm{Mi}^{1.5} + \mathrm{Ma}) \times \mathrm{L} \times \mathrm{S}^{0.5}/10 \leq \mathrm{P} \leq 50 \times \mathrm{Vt}^{(1/3)}$$

P indicates a thickness (mm) of a tube that connects the bubble trap container and the culture container,
S indicates a gas-liquid interface area ($m^2$) of a culture solution,
L indicates a length (m) of a tube that connects the bubble trap container and the culture container,
Mi indicates an aeration amount (L/min) from a microsparger having a pore diameter of 1 $\mu$m to 200 $\mu$m,
Ma indicates an aeration amount (L/min) of a macrosparger having a pore diameter of 0.1 mm to 10 mm, and
Vt indicates a bubble trap container capacity (L).

<11> The method for producing a product according to any one of <1> to <10>, in which the following expression is satisfied,

$$W^{0.5}/1{,}000 \leq E \leq Vc/S/100$$

in the expression,

E indicates a surface area ($m^2$) of an exhaust filter connected to the bubble trap container,
W indicates a culture solution amount (L) in the culture container,
Vc indicates a culture container volume (L), and
S indicates a gas-liquid interface area ($m^2$) of a culture solution.

<12> The method for producing a product according to any one of <1> to <11>, in which the following expression is satisfied,

$$1{,}000 \times S^2 \times Mi^2 \times Ma^{0.5} \times L/P^2/(Vc - W) \leq F \leq W/10$$

in the expression,

F indicates an upper surface aeration amount (L/min) flowing into the culture container,
Vc indicates a culture container volume (L),
W indicates a culture solution amount (L) in the culture container,
Mi indicates an aeration amount (L/min) from a microsparger having a pore diameter of 1 $\mu$m to 200 $\mu$m,
Ma indicates an aeration amount (L/min) of a macrosparger having a pore diameter of 0.1 mm to 10 mm,
L indicates a length (m) of a tube that connects the bubble trap container and the culture container,
P indicates a thickness (mm) of a tube that connects the bubble trap container and the culture container, and
S indicates a gas-liquid interface area ($m^2$) of a culture solution.

<13> The method for producing a product according to any one of <1> to <12>, in which the culture container is a single-use culture tank, and/or
the bubble trap container is a single-use bag.
<14> The method for producing a product according to any one of <1> to <13>, in which the bubble trap container is a container that is sealed except for a pipe connected to the outside.
<15> The method for producing a product according to any one of <1> to <14>, in which a viable cell density in the culture solution is $10 \times 10^6$ to $300 \times 10^6$ cells/mL.
<16> The method for producing a product according to any one of <1> to <15>, in which a culture solution amount in the culture container is 10 L or more.
<17> The method for producing a product according to any one of <1> to <16>, in which a method of culturing the cell is perfusion culture.
<18> The method for producing a product according to any one of <1> to <17>, in which the cell is a CHO cell.
<19> The method for producing a product according to any one of <1> to <18>, in which the product produced by the cell is an antibody.
<20> The method for producing a product according to any one of <1> to <19>, in which a culture period is 14 days or more.
<21> A cell culture device comprising:

a culture container accommodating a cell suspension containing cells; and
at least one or more bubble trap containers capable of accumulating bubbles, the one or more bubble trap containers being connected to an outside of the culture container by a pipe.

<22> The cell culture device according to <21>, in which at least one or more exhaust filters are connected to the bubble trap container.
<23> The cell culture device according to <21> or <22>, in which a waste liquid line is connected to the bubble trap container.
<24> The cell culture device according to any one of <21> to <23>, in which a discharge port for a gas which is aerated to the culture container is only a line that connects the culture container and the bubble trap container.

<25> The cell culture device according to any one of <21> to <24>, in which the waste liquid line connected to the bubble trap container is connected to a waste liquid tank maintaining an aseptic state.

<26> The cell culture device according to <25>, in which the waste liquid tank is allowed to be switched aseptically.

<27> The cell culture device according to any one of <21> to <26>, in which a physical anti-foaming mechanism is provided in the bubble trap container.

<28> The cell culture device according to any one of <21> to <27>, in which the following expression is satisfied,

$$Mi^2 \times Ma^{0.5}/S/200 \leq Vt \leq 10 \, W$$

in the expression,

Vt indicates a bubble trap container capacity (L),
Mi indicates an aeration amount (L/min) from a microsparger having a pore diameter of 1 $\mu$m to 200 $\mu$m,
Ma indicates an aeration amount (L/min) of a macrosparger having a pore diameter of 0.1 mm to 10 mm,
S indicates a gas-liquid interface area ($m^2$) of a culture solution, and
W indicates a culture solution amount (L) in the culture container.

<29> The cell culture device according to any one of <21> to <28>, in which the following expression is satisfied,

$$L \leq P^2/(Mi^{1.5} + Ma) \times S^{0.5}$$

in the expression,

L indicates a length (m) of a tube that connects the bubble trap container and the culture container,
S indicates a gas-liquid interface area ($m^2$) of a culture solution,
P indicates a thickness (mm) of a tube that connects the bubble trap container and the culture container,
Mi indicates an aeration amount (L/min) from a microsparger having a pore diameter of 1 $\mu$m to 200 $\mu$m, and
Ma indicates an aeration amount (L/min) of a macrosparger having a pore diameter of 0.1 mm to 10 mm.

<30> The cell culture device according to any one of <21> to <29>, in which the following expression is satisfied,

$$(Mi^{1.5} + Ma) \times L \times S^{0.5}/10 \leq P \leq 50 \times Vt^{(1/3)}$$

P indicates a thickness (mm) of a tube that connects the bubble trap container and the culture container,
S indicates a gas-liquid interface area ($m^2$) of a culture solution,
L indicates a length (m) of a tube that connects the bubble trap container and the culture container,
Mi indicates an aeration amount (L/min) from a microsparger having a pore diameter of 1 $\mu$m to 200 $\mu$m,
Ma indicates an aeration amount (L/min) of a macrosparger having a pore diameter of 0.1 mm to 10 mm, and
Vt indicates a bubble trap container capacity (L).

<31> The cell culture device according to any one of <21> to <30>, in which the following expression is satisfied,

$$W^{0.5}/1,000 \leq E \leq Vc/S/100$$

in the expression,

E indicates a surface area E ($m^2$) of an exhaust filter connected to the bubble trap container,
W indicates a culture solution amount (L) in the culture container,
Vc indicates a culture container volume (L), and
S indicates a gas-liquid interface area ($m^2$) of a culture solution.

<32> The cell culture device according to any one of <21> to <31>, in which a volume of the culture container is 10 L or more.

<33> The cell culture device according to any one of <21> to <32>, in which the culture container is a single-use culture tank, and/or
the bubble trap container is a single-use bag.
<34> The cell culture device according to any one of <21> to <33>, in which the bubble trap container is a container that is sealed except for a pipe connected to the outside.

Effect of the invention

According to the present invention, it is possible to suppress the clogging of an exhaust filter due to bubbles generated on an upper surface of a culture solution and to continue the cell culture at a high concentration.

BRIEF DESCRIPTION OF THE DRAWINGS

Fig. 1 shows an example of a cell culture device according to an embodiment of the present invention.
Fig. 2 shows another example of the cell culture device according to the embodiment of the present invention.
Fig. 3 shows still another example of the cell culture device according to the embodiment of the present invention.
Fig. 4 shows still another example of the cell culture device according to the embodiment of the present invention.

DESCRIPTION OF THE PREFERRED EMBODIMENTS

Hereinafter, the contents of the present invention will be described in detail. In the present specification, a numerical value range indicated using "to" means a range including numerical values described before and after "to" as a minimum value and a maximum value, respectively.

A method for producing a product according to the embodiment of the present invention is a method including culturing a cell using a cell culture device to produce a product from the cell. The cell culture device that is used in the method for producing a product according to the embodiment of the present invention is a device that includes a culture container accommodating a cell suspension containing cells and at least one or more bubble trap containers capable of accumulating bubbles, the one or more bubble trap containers being connected to an outside of the culture container.

The cell culture device according to the embodiment of the present invention is a device that includes a culture container accommodating a cell suspension containing cells and at least one or more bubble trap containers capable of accumulating bubbles, the one or more bubble trap containers being connected to an outside of the culture container by a pipe.

In the present invention, since a bubble trap container is installed between a culture container (also referred to as a culture tank) accommodating a cell suspension containing cells and an exhaust filter installed in an upper part of the culture container, it is possible to retain bubbles in the bubble trap container, which makes it possible to prevent the bubbles from clogging the exhaust filter and to continue the culture.

In the related art, a line other than the line of the exhaust filter is installed to be positively sucked out bubbles. However, there is a problem that in a case where bubbles more than expected are generated, the bubbles reach the exhaust filter, which causes the clogging of the exhaust filter. In addition, since it is difficult to prepare a sufficient space even in a case where a space for bubble trap for accumulating bubbles is installed in the culture container, the space is immediately filled with bubbles, and the bubble reaches the exhaust filter. Further, in a case where a line for discharging bubbles is simply attached to the culture container, the pressure inside the culture container increases due to a pressure loss between the bubbles flowing into the line and the line, and in general, an excessive pressure prevention mechanism that is installed in a culture device works. Then, the supply of various gases to the inside of the culture container is stopped, and the culture cannot be continued.

On the other hand, in the present invention, the bubbles directed to the exhaust filter can be reliably trapped by providing a space for trapping bubbles in front of the exhaust filter.

In addition, in a case where a waste liquid line is provided in the bubble trap container, the bubble trap container can be prevented from being filled with bubbles by discharging the bubbles from the waste liquid line even in a case where the space of the bubble trap container is likely to be filled with bubbles and a liquid generated from the bubbles, which makes it possible to prevent the exhaust filter from being clogged by the bubbles.

Further, in a case where a line (tube) connecting the culture container and a container that traps bubbles is made to have an appropriate length and an appropriate thickness, it is possible to suppress the pressure loss caused by the flow of the bubbles, and it is possible to continue the culture without operating the excessive pressure prevention mechanism and without stopping the supply of various gases to the inside of the culture tank.

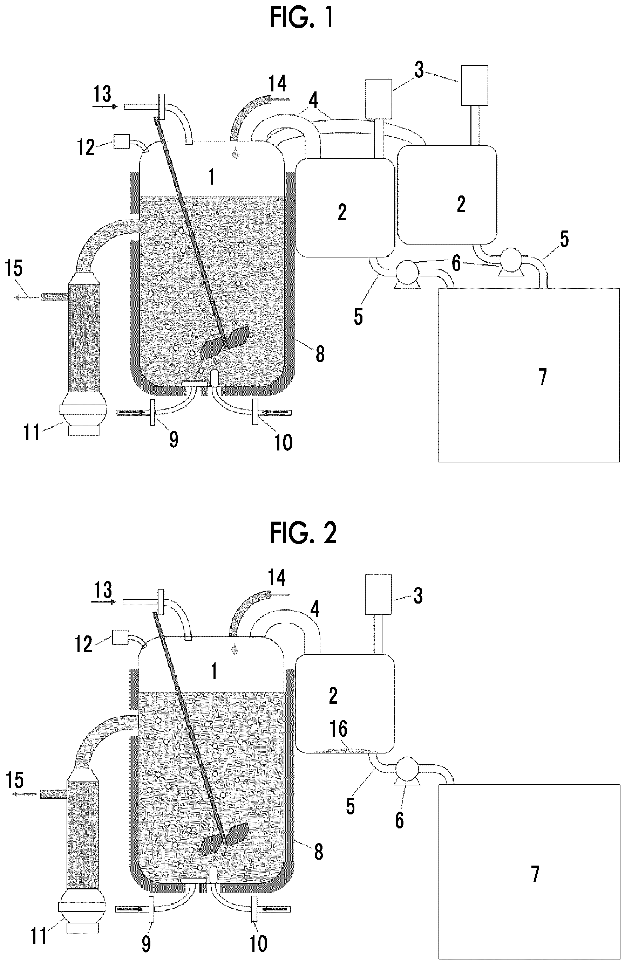

Specific examples of the cell culture device according to the embodiment of the present invention are shown in Fig. 1 to Fig. 4.

In Fig. 1 to Fig. 4, a culture container 1 is installed inside a vessel 8. The culture container 1 is a container that accommodates a cell suspension containing cells. A stirring device having a stirring blade may be provided inside the culture container 1. In a case where a stirring blade is rotated, the culture medium accommodated in the culture container 1 together with cells is stirred, and thus the homogeneity of the medium is maintained. The culture container 1 may be a single-use culture tank (single-use bag), and it is preferably a single-use bag made of a material having low elution properties or a material having gas barrier properties.

A bubble trap container 2 is connected to the outside of the culture container 1 by a tube 4 that is a pipe. The material of the tube 4 is not particularly limited; however, it is preferably silicone or a material that can be subjected to thermal fusion welding. In Fig. 1, although two bubble trap containers 2 are connected, the number of the bubble trap containers 2 is not particularly limited, and it may be one or may be two or two or more. Fig. 2 to Fig. 4 show a case where the number of the bubble trap containers 2 is one. It is preferable that a discharge port for a gas, which is aerated to the culture container 1, is only the line (the tube 4) that connects the culture container 1 and the bubble trap container.

At least one or more exhaust filters 3 are connected to the bubble trap container 2.

A waste liquid line 5 is connected to the bubble trap container 2, and a waste liquid pump 6 is installed in the middle of the waste liquid line 5. The waste liquid line 5 connected to the bubble trap container 2 is connected to a waste liquid tank 7 maintaining an aseptic state. The waste liquid tank may be configured to be capable of being switched aseptically.

It is preferable that the bubble trap container 2 is a container that is sealed except for a pipe connected to the outside. The bubble trap container may be a single-use bag, and it is preferably a single-use bag made of a material having low elution properties or a material having gas barrier properties.

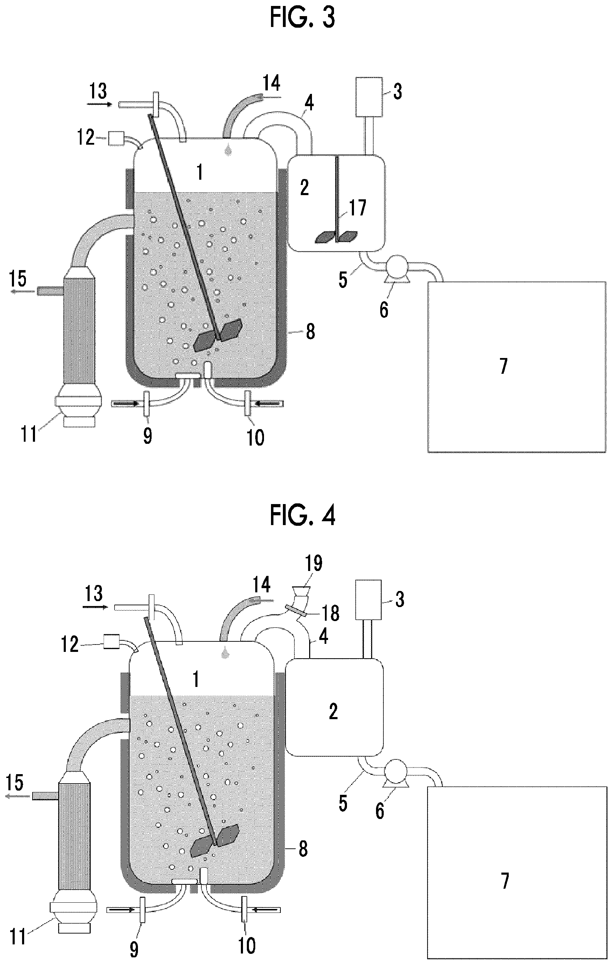

Fig. 2 shows a state in which an anti-foaming agent 16 is added into the bubble trap container 2. Fig. 3 shows a state in which a physical anti-foaming mechanism 17 is provided in the bubble trap container 2. As the physical anti-foaming mechanism 17, a stirring device or the like can be used.

Fig. 4 shows a case where a tube having a branch is used as the tube that connects the culture container 1 and the bubble trap container 2. A tube clamp 18 is provided in the branched portion, and an aseptic connector 19 is provided at a terminal of the branched portion, where an additional bubble trap container is configured to be connectable thereto.

In Fig. 1 to Fig. 4, a macrosparger 9 and a microsparger 10 are provided below the culture container 1. The macrosparger 9 is used for adjusting the gas concentration in the culture solution, and it is preferably a sparger for adjusting the $CO_2$ concentration in the culture solution. The microsparger 10 is used for adjusting the gas concentration in the culture solution, and it is preferably a sparger for adjusting the oxygen concentration in the culture solution. A perfusion device 11 is connected to the culture container 1, a culture solution inside the culture container 1 passes through the perfusion device 11, and a permeated liquid 15 containing a product of cells is taken out from the culture container 1.

The culture container 1 is equipped with a pressure sensor 12 for measuring the pressure in the space of the upper part of the culture solution in a case where the culture solution is introduced into the culture container. An opening for carrying out upper surface aeration 13 is provided in the upper part of the culture container 1. In addition, an opening for introducing a supply culture medium 14 is provided in the upper part of the culture container 1.

In the method for producing a product and the cell culture device according to the embodiment of the present invention, it is preferable that one or more of the following expression 1 to expression 5 are satisfied.

$$(\text{Expression 1}) \ \text{Mi}^2 \times \text{Ma}^{0.5}/\text{S}/200 \leq \text{Vt} \leq 10 \ \text{W}$$

$$(\text{Expression 2}) \ \text{L} \leq [\text{P}^2/(\text{Mi}^{1.5} + \text{Ma})] \times \text{S}^{0.5}$$

$$(\text{Expression 3}) \ (\text{Mi}^{1.5} + \text{Ma}) \times \text{L} \times \text{S}^{0.5}/10 \leq \text{P} \leq 50 \times \text{Vt}^{(1/3)}$$

$$(\text{Expression 4}) \ \text{W}^{0.5}/1{,}000 \leq \text{E} \leq \text{Vc}/\text{S}/100$$

$$(\text{Expression 5}) \ 1{,}000 \times \text{S}^2 \times \text{Mi}^2 \times \text{Ma}^{0.5} \times \text{L}/\text{P}^2/(\text{Vc} - \text{W}) \leq \text{F} \leq \text{W}/10$$

In a case where P is set in the range defined by Expression 3, it is possible to obtain an effect of suppressing the pressure (see Example 9 and Example 13 described later).

In a case where F is set in the range defined by Expression 5, it is possible to obtain an effect of suppressing an increase in pressure (see Example 12 and Example 13, which show cases outside the range of Expression 5).

In the expression,

Vc indicates a culture container volume (L), and

W indicates a culture solution amount (L) in the culture container.

S indicates a gas-liquid interface area ($m^2$) of a culture solution, and

F indicates an upper surface aeration amount (L/min) flowing into the culture container.

E indicates a surface area ($m^2$) of an exhaust filter connected to the bubble trap container,

Mi indicates an aeration amount (L/min) from a microsparger having a pore diameter of 1 $\mu$m to 200 $\mu$m,

Ma indicates an aeration amount (L/min) of a macrosparger having a pore diameter of 0.1 mm to 10 mm,

Vt indicates a bubble trap container capacity (L),

L indicates a length (m) of a tube that connects the bubble trap container and the culture container, and

P indicates a thickness (mm) of a tube that connects the bubble trap container and the culture container.

The method of measuring each parameter will be described below.

S (gas-liquid interface area of culture solution): This is a projected area in a case where cutting is carried out horizontally at the upper end of the solution surface in a case where filling is carried out with a solution amount at the time of culture, and it can be measured using a laser measuring instrument or a caliper.

W (culture solution amount in culture container): This can be measured by a weight measuring device attached to the culture device. In a case of not being attached thereto, it can also be measured by adding water until a height at the time of culture is reached and then separately measuring the weight of the water.

L (length of tube): This can be measured by an appropriate length measuring instrument such as a ruler, a convex, a caliper, or the like.

P (thickness of tube): This is according to the catalog specification or can be measured by a caliper.

Vc (culture container volume): This is according to the catalog specification or can be calculated from a specific gravity of a fluid in a case where filling is carried out with an appropriate fluid.

Vt (bubble trap container capacity): This is according to the catalog specification or can be calculated from a specific gravity of a fluid in a case where filling is carried out with an appropriate fluid.

E (surface area of exhaust filter): This is according to the catalog specification.

F (upper surface aeration amount that flows into culture container), Mi (aeration amount from microsparger), and Ma (aeration amount of macrosparger): These are set values of aeration amounts in the sparger.

From the viewpoint of large-scale culture and productivity, Vc (the culture container volume) is preferably 10 L to 50,000 L, more preferably 20 L to 20,000 L, still more preferably 100 L to 2,000 L, and particularly preferably 300 to 1,500 L.

W (the culture solution amount in the culture container) is preferably 10 L to 20,000 L, more preferably 20 L to 5,000 L, still more preferably 100 L to 2,000 L, and particularly preferably 300 to 1,500 L.

The S (the gas-liquid interface area of the culture solution) is preferably 0.01 $m^2$ to 10 $m^2$, and more preferably 0.05 $m^2$ to 5 $m^2$.

F (the upper surface aeration amount that flows into the culture container) is preferably 0.01 L/min to 200 L/min, and more preferably 0.1 L/min to 100 L/min.

E (the surface area of the exhaust filter connected to the bubble trap container) is preferably 0.01 $m^2$ to 10 $m^2$, and more preferably 0.02 $m^2$ to 5 $m^2$.

Mi (the aeration amount from a microsparger having a pore diameter of 1 $\mu$m to 200 $\mu$m) is preferably 0.1 L/min to 100 L/min, and more preferably 0.5 L/min to 50 L/min. The pore diameter of the microsparger is preferably 5 $\mu$m to 100 $\mu$m, and more preferably 10 $\mu$m to 50 $\mu$m. Mi may be an aeration amount from a microsparger for maintaining the oxygen concentration in the culture solution.

Ma (the aeration amount of a macrosparger having a pore diameter of 0.1 mm to 10 mm) is preferably 0.01 L/min to 100 L/min, and more preferably 0.1 L/min to 50 L/min. The pore diameter of the macrosparger is preferably 0.2 mm to 5 mm, and more preferably 0.5 mm to 2 mm. Ma may be an aeration amount of a macrosparger for adjusting the $CO_2$ concentration in the culture solution.

Vt (the bubble trap container capacity) is preferably 0.5 L to 1,000 L, and more preferably 1 L to 500 L.

L (the length of the tube that connects the bubble trap container and the culture container) is preferably 0.05 m to 5 m, and more preferably 0.1 m to 3 m.

P (the thickness of the tube that connects the bubble trap container and the culture container) is preferably 2 mm to 300 mm, and more preferably 5 mm to 50 mm.

In the present invention, the viable cell density in the culture solution is preferably $10 \times 10^6$ to $300 \times 10^6$ cells/mL, more preferably $30 \times 10^6$ to $300 \times 10^6$ cells/mL, still more preferably $40 \times 10^6$ to $300 \times 10^6$ cells/mL, still more preferably $50 \times 10^6$ to $300 \times 10^6$ cells/mL, still more preferably $0 \times 10^6$ to $300 \times 10^6$ cells/mL still more preferably $100 \times 10^6$ to $300 \times 10^6$ cells/mL, and even still more preferably $120 \times 10^6$ to $300 \times 10^6$ cells/mL. M may be used to denote $10^6$.

In the cell culture, an anti-foaming agent may be added to the culture medium. The anti-foaming component of the anti-foaming agent is preferably silicone-based, and it is particularly preferably dimethicone. The anti-foaming component of the anti-foaming agent is preferably an anti-foaming component containing polydimethylsiloxane, and it is more preferably an anti-foaming component in which fine powder silica is contained in polydimethylsiloxane. As the anti-foaming agent, it is possible to use, for example, HyClone ADCF Antifoam Agent manufactured by Cytiva. In addition, since it is not necessary to consider cell damage due to the anti-foaming agent, an anti-foaming agent having a higher concentration than the above-described anti-foaming agent can also be used.

In addition, as shown in Fig. 2, the above-described anti-foaming agent can be used as the anti-foaming agent 16 in a case where the anti-foaming agent 16 is added into the bubble trap container 2.

In the present invention, a cell suspension extracted from the culture container may be allowed to pass through a separation membrane to separate the cell suspension into a cell-containing liquid and a permeated liquid. This operation can be carried out using a perfusion device. In this operation, the cell suspension extracted from the culture tank is separated into a cell-containing liquid having a cell concentration higher than that of the cell suspension and a permeated liquid having a cell concentration lower than that of the cell suspension. The cell concentration can be measured by a live/dead cell analyzer Vi-CELL XR, manufactured by Beckman Coulter Life Sciences.

The membrane separation treatment step described above is preferably tangential filtration, more preferably alternating tangential flow (ATF) or tangential flow, and most preferably alternating tangential flow. Examples of the filter capable of carrying out the alternating tangential flow include SuATF 10-S02PES or F2 RF02PES, manufactured by Repligen Corporation.

As the culture medium that is used for cell culture, a culture medium that is generally used for culturing animal cells can be used. For example, CD OptiCHO (manufactured by Thermo Fisher Scientific, Inc.), Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

Although the pH of the culture medium varies depending on the cells to be cultured, the culture medium generally has a pH of 6.0 to 8.0, preferably has a pH of 6.4 to 7.6, and more preferably has a pH of 6.7 to 7.4.

The culture temperature is generally 30°C to 40°C, preferably 32°C to 39°C, and more preferably 36°C to 38°C, and the culture temperature may be changed during the culture.

The culture can be preferably carried out in an atmosphere having a $CO_2$ concentration of 0% to 40%, preferably 2% to 25%, and still more preferably 3% to 20%.

The culture period is not particularly limited; however, it is generally 12 hours to 90 days, preferably 1 day to 80 days, more preferably 1 day or more and less than 70 days, still more preferably 5 days or more and less than 65 days, and particularly preferably 7 days or more and less than 60 days. In the present invention, the culture period is preferably 14 days or more.

In the culture, the culture medium can be replaced, aerated, and stirred as necessary. In a case of carrying out stirring of the culture, the rotation speed of stirring is not particularly limited; however, the stirring power per unit volume is generally 10 to 300 kW/m$^3$, preferably 20 to 200 kW/m$^3$, and more preferably 30 to 100 kW/m$^3$.

The cell culture can be carried out using a cell culture device having the configuration described above in the present specification. The cell culture device may be any one of a fermenter tank type culture device, an air lift type culture device, a culture flask type culture device, a spinner flask type culture device, a microcarrier type culture device, a fluidized bed type culture device, a hollow fiber type culture device, a roller bottle type culture device, a filling tank type culture device, or the like.

The viscosity of the culture solution is preferably 1.2 mPa·s or more and less than 15 mPa s, more preferably 1.4 mPa s or more and less than 12 mPa s, and particularly preferably 1.6 mPa s or more and less than 10 mPa s.

In the present invention, a pH adjusting agent may be added during the culture. From the viewpoint of the clogging of the filtration filter flow channel, a pH adjusting agent to be added to the culture tank is such that an adding amount thereof per day is preferably 8 mmol/day/L or less and more preferably 7 mmol/day/L or less. Here, it is not necessary to add a pH adjusting agent.

The pH adjusting agent is not particularly limited; however, it is preferably an aqueous $Na_2CO_3$ solution, an aqueous NaOH solution, or an aqueous $NaHCO_3$ solution, and it is more preferably an aqueous $NaHCO_3$ solution. The pH adjusting agent may be added alone or may be added by being mixed with a culture medium or an anti-foaming agent. In order to avoid the local fluctuation in pH in the culture solution due to the addition of the pH adjusting agent, it is preferable that the pH adjusting agent is added by being mixed with a culture medium.

The mode of the method of culturing cells is not particularly limited, and the culture method may be, for example, any one of perfusion culture, batch culture, or fed-batch culture, where perfusion culture is preferable.

The batch culture is a discontinuous method in which cells are proliferated for a short period in a culture solution with a fixed volume and then completely harvested.

The fed-batch culture is a culture method that improves the batch process by a bolus supply or continuous supply of a culture medium, followed by replenishing the consumed components of the culture medium.

The perfusion culture is a culture method in which a fresh culture medium is added and concurrently, a used culture medium is removed, and thus it provides a possibility that the batch culture and the fed-batch culture can be further improved. In general, the perfusion culture makes it possible to achieve a high viable cell density. A typical perfusion culture begins with a batch culture start-up lasting 1 or 2 days, thereafter a fresh supply culture medium is added to the culture product continuously, stepwise, and/or intermittently, and the used culture medium is removed at the same time. In the perfusion culture, methods such as sedimentation, centrifugation, and filtration can be used to remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture product and the used culture medium that is removed may substantially not include cells or may have much fewer cells than the culture product. A product that is expressed by cell culture can be retained in the culture product or harvested by the selection of the membrane pore diameter. To prevent the viable cell density during the culture from becoming excessive, a part of the culture solution may be extracted together with the cells, and the same amount of a fresh culture medium may be added to reduce the viable cell density (cell bleeding).

The kind of the cell in the present invention is not particularly limited; however, examples thereof include eukaryotic cells such as an animal cell, a plant cell, and yeast, prokaryotic cells such as Bacillus subtilis, and Escherichia coli. The cell is preferably an animal cell (more preferably a mammalian cell) or an insect cell, and it is most preferably a mammalian cell. The cell may be a primary cell or a cell established as a cell line.

Examples of the cell include a Chinese hamster ovary (CHO) cell, a HEK cell (a cell derived from the human embryonic kidney), a BHK cell, a 293 cell, a myeloma cell (such as an NS0 cell), a PerC6 cell, a SP2/0 cell, a hybridoma cell, a COS cell (a cell derived from the kidney of the African green monkey), a 3T3 cell, a HeLa cell, a Vero cell (a renal epithelial cell of the African green monkey), a MDCK cell (a cell derived from a canine kidney renal tubular epithelial cell), a PC12 cell, and a WI38 cell. Among these, a CHO cell, a HEK cell, a BHK cell, or a hybridoma is preferable, where a CHO cell or a HEK cell is more preferable, and a CHO cell is most preferable. The CHO cell is widely used for the production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

It is preferable that the cell viability is high; however, it is preferably 85% or more, more preferably 90% or more, particularly preferably 95% or more, and most preferably 99% or more.

These cells may be cells into which a foreign gene encoding a protein desired to be expressed has been introduced. An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells. Examples thereof include a promoter of the immediate early (IE) gene of the cytomegalovirus (CMV), an early promoter of SV40, a retrovirus promoter, a metallothionein promoter, a heat shock promoter, an SRα promoter, and a promoter and enhancer of the Moloney murine leukemia virus. In addition, an enhancer of the IE gene of human CMV may be used together with the promoter.

As the selection marker gene, it is possible to use, for example, a drug resistance gene (a neomycin resistance gene, a DHFR gene, a puromycin resistance gene, a blasticidin resistance gene, a hygromycin resistance gene, a cycloheximide resistance gene), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

The method for producing a product according to the embodiment of the present invention includes culturing a cell using a cell culture device to produce a product from the cell.

In the present invention, the kind of product is not particularly limited; however, the product is preferably a recombinant protein. Examples of the product include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv). In addition, it may be, in addition to the above, an adenovirus, an adeno-associated virus, a lentivirus, or the like.

The product is preferably an antibody, and more preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of a non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culture by culturing a recombinant cell transformed with the above vector and expressing the incorporated DNA.

The bispecific antibody is an antibody that recognizes two kinds of antigenic specificity, which are different from each other. Various forms of bispecific antibodies are present. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other. In addition, a bispecific antibody can be expressed by introducing a gene encoding the bispecific antibody into a cell.

The Fc fusion protein indicates a protein having an Fc region and includes an antibody. The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

The antibody is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

It is possible to purify a product that is produced by the above-described culture. For the separation and purification of the product, the separation and purification methods that are used for general proteins may be used. For example, it is possible to carry out separation and purification of a product by appropriately selecting and combining means such as chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the product obtained as above can be measured according to absorbance measurement, enzyme-linked immunosorbent assay (ELISA), or the like.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify the productor partially remove a peptide thereof by allowing an appropriate polypeptide modifying enzyme to act on the useful substance before or after purification. As the polypeptide modifying enzyme, for example, trypsin, chymotrypsin, lysyl endopeptidase, protein kinase, or glucosidase is used.

The present invention will be more specifically described using the following examples; however, it is not limited by the examples.

Examples

<Establishment of antibody-producing cell>

A vector containing a nucleic acid sequence encoding each of IgG1 and IgG4 was constructed, and the constructed vector was introduced into a CHO-DG44 cell to prepare a CHO-DG44 cell expressing IgG1 (an IgG1 cell) and a CHO-DG44 cell expressing IgG4 (an IgG4 cell). The construction of the vector and the introduction thereof into the cell were carried out according to Example 2 of JP2016-517691A. As described above, CHO cells producing monoclonal antibodies were prepared and used in Examples below.

In the following Examples and Comparative Examples, experiments were carried out using the cell culture device shown in Fig. 1.

<Examples 1 to 3>

Cell: CHO cell
Culture method: perfusion culture
Culture medium: CD OptiCHO (manufactured by Thermo Fisher Scientific, Inc.)
Culture tank: A plastic single-use culture container having a diameter of 349 mm and a tank height of 685 mm, where the stirring blade is a propeller blade having a diameter of 116 mm
Culture solution amount: 50 L
Seeding density of viable cells: $5 \times 10^5$ cells/mL
Culture environment: 36°C or higher and 38°C or lower, a pH of 6.7 or more and 7.4 or less, and a $CO_2$ concentration of 160 mmHg or lower

Viable cell density to be reached: $1.2 \times 10^8$ cells/mL

After the cell seeding, the cell density was measured once a day, the culture solution was extracted so that the cell density was the above-described cell density to be reached in a case of exceeding the above-described cell density to be reached, the same amount of the culture medium was added thereto to adjust the total culture solution amount to be constant, and an adjustment was carried out so that the cell density was maintained at the density to be reached.

Perfusion ratio: 1.2

Perfusion was started 2 days after the start of the culture. That is, a new culture medium was continuously supplied to the culture tank, and at the same time, a permeated liquid having an amount that was the same as that of the supply culture medium was extracted from the filtration filter. Then, the amounts of the new culture medium to be supplied and the permeated liquid to be extracted were adjusted to be 1.2 times the culture solution amount per day.

Filtration: A F4 RF02PES-V2 membrane was used in a system of ATF4, manufactured by Repligen Corporation. Regarding the F4 RF02PES-V2 membrane, the pore diameter is 0.2 μm, the hollow fiber diameter is 1 mm, and the filtration area is 0.77 m$^2$.

Oxygen concentration: The oxygen concentration is maintained at 33% or more.

Anti-foaming agent: The addition of HyClone ADCF Antifoam Agent, manufactured by Cytiva, was continuously started from the 7th day of culture at an addition rate of 0.2 mg/L/hr in terms of Simeticon, as an active ingredient.

Culture period: In Examples 1 to 3, the culture was carried out for 34 days.

<Example 4>

Cell: CHO cell
Culture method: Fed-batch culture
Culture medium: CD OptiCHO (manufactured by Thermo Fisher Scientific, Inc.)
Culture tank: A plastic single-use culture container having a diameter of 349 mm and a tank height of 685 mm, where the stirring blade is a propeller blade having a diameter of 116 mm
Culture solution amount: 50 L
Seeding density of viable cells: $5 \times 10^5$ cells/mL
Culture environment: 36°C or higher and 38°C or lower, a pH of 6.7 or more and 7.4 or less, and a CO2 concentration of 160 mmHg or lower

Viable cell density to be reached: $4.0 \times 10^7$ cells/mL, after the cell seeding, the cell density was measured once a day.

Oxygen concentration: The oxygen concentration was maintained at 33% or more.

Anti-foaming agent: HyClone ADCF Antifoam Agent, manufactured by Cytiva, was appropriately added as necessary.

Culture period; in Example 4, the culture was carried out for 14 days.

<Examples 5 to 16 and Comparative Example 1>

Culture tank: A plastic single-use culture container having a diameter of 756 mm and a tank height of 1,472 mm, where the stirring blade is a propeller blade having a diameter of 251 mm.

Simulated solution: A solution obtained by dissolving 7 g of NaCl, 2 g of NaHCO$_3$, and 1 g of surfactant Kolliphor P188 in 1 L with pure water
Liquid amount: 500 L
Perfusion ratio: Perfusion was simulatedly carried out at a perfusion ratio of 1.2.
Filtration: A suATF10-S02PES membrane was used in a system of ATF10, manufactured by Repligen Corporation. Regarding the suATF10-S02PES membrane, the pore diameter is 0.2 $\mu$m, the hollow fiber diameter is 1 mm, and the filtration area is 11 m$^2$.
Anti-foaming agent in bubble trap container: HyClone ADCF Antifoam Agent, manufactured by Cytiva
In Comparative Example 1, a device in which a bubble trap container was provided in a culture tank, like the device described in JP2018-19615A, was used.

<Measurement of maximum internal pressure>

A TruTorr II pressure sensor (Thermo Fisher Scientific, Inc.) was aseptically connected to a tube extending from a plastic single-use bag, and the pressure inside the single-use bag was measured.

The contents and results of Examples 1 to 16 and Comparative Example 1 are shown in the following table.

The numerical value range in parentheses in the column of the upper surface aeration amount indicates a range that satisfies the following expression.

$$(\text{Expression 5}) \ 1,000 \times S^2 \times Mi^2 \times Ma^{0.5} \times L/P^2/(Vc - W) \leq F \leq W/10$$

The numerical value range in parentheses in the column of the area of the exhaust filter indicates that satisfies a range of the following expression.

$$(\text{Expression 4}) \ W^{0.5}/1,000 \leq E \leq Vc/S/100$$

The numerical value range in parentheses in the column of the bubble trap container capacity indicates a range that satisfies the following expression.

$$\text{(Expression 1) } Mi^2 \times Ma^{0.5}/S/200 \leq Vt \leq 10\ W$$

The numerical value range in parentheses in the column of the tube length indicates a range that satisfies the following expression.

$$\text{(Expression 2A) } (S/\pi)^{0.5}/2 \leq L \leq [P^2/(Mi^{1.5} + Ma)] \times S^{0.5}$$

The numerical value range in parentheses in the column of the tube thickness indicates a range that satisfies the following expression.

$$\text{(Expression 3) } (Mi^{1.5} + Ma) \times L \times S^{0.5}/10 \leq P \leq 50 \times Vt^{(1/3)}$$

A to D in the results in Table 3 were determined according to the following criteria.

A: A case where the maximum internal pressure is less than 5 psi
B: A case where although the maximum internal pressure reaches 5 psi instantaneously (only once in the pressure display device), the culture can be continued
C: A case where although the maximum internal pressure exceeds 5 psi intermittently (2 times or more consecutively in the pressure display device), and the culture control is stopped temporarily, the culture can be continued
D: A case where bubbles are accumulated in the exhaust filter, and the culture cannot be continued

[Table 1]

| Example Comparative Example | Liquid | Culture method | Viable cell density M cells/mL | Culture tank volume L | Culture solution volume L | Gas-liquid interface area m² | Upper surface aeration amount L/min | Exhaust filter of culture tank |
|---|---|---|---|---|---|---|---|---|
| Example 1 | Culture solution | Perfusion | 120 | 65.5 | 50 | 0.096 | 0.5 (0.19~5) | Absent |
| Example 2 | Culture solution | Perfusion | 200 | 65.5 | 50 | 0.096 | 1 (0.74~5) | Absent |
| Example 3 | Culture solution | Perfusion | 120 | 65.5 | 50 | 0.096 | 0.5 (0.33~5) | Absent |
| Example 4 | Culture solution | Fed-batch | 40 | 65.5 | 50 | 0.096 | 0.5 (0.03~5) | Absent |
| Example 5 | Simulated solution | Perfusion | | 660 | 500 | 0.438 | 10 (2.51~50) | Absent |
| Example 6 | Simulated solution | Perfusion | | 660 | 500 | 0.438 | 10 (1.41~50) | Absent |
| Example 7 | Simulated solution | Perfusion | | 660 | 500 | 0.438 | 5 (2.51~50) | Absent |
| Example 8 | Simulated solution | Perfusion | | 660 | 500 | 0.438 | 20 (2.51~50) | Absent |
| Example 9 | Simulated solution | Perfusion | | 660 | 500 | 0.438 | 10 (2.51~50) | Absent |
| Example 10 | Simulated solution | Perfusion | | 660 | 500 | 0.438 | 10 (2.51~50) | Absent |
| Example 11 | Simulated solution | Perfusion | | 660 | 500 | 0.438 | 10 (2.51~50) | Absent |
| Example 12 | Simulated solution | Perfusion | | 660 | 500 | 0.438 | 10 (10.03~50) | Absent |
| Example 13 | Simulated solution | Perfusion | | 660 | 500 | 0.438 | 10 (10.04~50) | Absent |

(continued)

| Example ComparativeExample | Liquid | Culture method | Viable cell density M cells/mL | Culture tank volume L | Culturesolution volume L | Gas-liquid interface area m² | Upper surface aeration amount L/min | Exhaust filter of culture tank |
|---|---|---|---|---|---|---|---|---|
| Example 14 | Simulated solution | Perfusion | | 660 | 500 | 0.438 | 10 (6.28~50) | Absent |
| Example 15 | Simulated solution | Perfusion | | 660 | 500 | 0.438 | 10 (2.51~50) | Present |
| Example 16 | Simulated solution | Perfusion | | 660 | 500 | 0.438 | 10 (0.03~50) | Present |
| Comparative Example 1 | Simulated solution | Perfusion | | 660 | 500 | 0.438 | 10 | Absent |

[Table 2]

| Example/ Comparative Example | Area of exhaust filter | Aeration amount of microsparger | Aeration amount of macrosparger | Rotation speed of stirring | Position of bubble trap container | Bubble trap container capacity | Number of bubble trap containers |
|---|---|---|---|---|---|---|---|
| | m² | L/min | L/min | rpm | | L | |
| Example 1 | 0.073 (0.007~6.85) | 4 | 1 | 250 | Outside | 10 (0.8~500) | 1 |
| Example 2 | 0.073 (0.007~6.85) | 10 | 2 | 250 | Outside | 10 (7.4~500) | 1 |
| Example 3 | 0.073 (0.007~6.85) | 8 | 1 | 250 | Outside | 10 (3.3~500) | 1 |
| Example 4 | 0.073 (0.007~6.85) | 1 | 0.2 | 250 | Outside | 10 (0.0~500) | 1 |
| Example 5 | 0.73 (0.022~15.06) | 20 | 10 | 150 | Outside | 100 (14.4~5000) | 2 |
| Example 6 | 0.73 (0.022~15.06) | 20 | 10 | 150 | Outside | 100 (14.4~5000) | 2 |
| Example 7 | 0.73 (0.022~15.06) | 20 | 10 | 150 | Outside | 100 (14.4~5000) | 2 |
| Example 8 | 0.73 (0.022~15.06) | 20 | 10 | 150 | Outside | 100 (14.4~5000) | 2 |
| Example 9 | 0.73 (0.022~15.06) | 20 | 10 | 150 | Outside | 100 (14.4~5000) | 2 |
| Example 10 | 0.73 (0.022~15.06) | 20 | 10 | 150 | Outside | 100 (14.4~5000) | 2 |
| Example 11 | 0.73 (0.022~15.06) | 20 | 10 | 150 | Outside | 100 (14.4~5000) | 2 |
| Example 12 | 0.73 (0.022~15.06) | 20 | 10 | 150 | Outside | 100 (14.4~5000) | 2 |
| Example 13 | 0.73 (0.022~15.06) | 40 | 10 | 150 | Outside | 100 (57.7~5000) | 2 |

| Example/ Comparative Example | Area of exhaust filter | Aeration amount of microsparger | Aeration amount of macrosparger | Rotation speed of stirring | Position of bubble trap container | Bubble trap container capacity | Number of bubble trap containers |
|---|---|---|---|---|---|---|---|
| | m² | L/min | L/min | rpm | Outside | L | |
| Example 14 | 0.73 (0.022~15.06) | 20 | 10 | 150 | Outside | 100 (14.4~5000) | 2 |
| Example 15 | 0.73 (0.022~15.06) | 20 | 10 | 150 | Outside | 100 (14.4~5000) | 2 |
| Example 16 | 0.73 (0.022~15.06) | 2 | 10 | 150 | Outside | 100 (0.1~5000) | 2 |
| Comparative Example 1 | 0.73 (0.022~15.06) | 20 | 10 | 150 | Inside | 20 (14.4~5000) | 1 |

[Table 3]

| Example/ Comparative Example | Tube length | Tube thickness | Tube material | Tube branch | Anti-foaming function | Waste liquid line | Maximum internal pressure | Result |
|---|---|---|---|---|---|---|---|---|
| | m | mm | | | | | psi | |
| Example 1 | 0.8 (0.09~1.39) | 6.35 (2.32~107.72) | Silicone | Present | Absent | Present | 2.9 | A |
| Example 2 | 0.8 (0.09~0.84) | 9.53 (8.72~107.72) | Silicone | Present | Absent | Present | 5 | B |
| Example 3 | 0.8 (0.09~1.19) | 9.53 (6.11~107.72) | Silicone | Present | Absent | Present | 4.5 | A |
| Example 4 | 0.8 (0.09~1.66) | 2.54 (0.31~107.72) | Silicone | Present | Absent | Present | 1.2 | A |
| Example 5 | 0.6 (0.19~2.42) | 19.05 (9.01~232.08) | Thermal fusion | Absent | Anti-foaming agent | Present | 4.5 | A |
| Example 6 | 0.6 (0.19~4.29) | 25.4 (9.01~232.08) | Thermal fusion | Absent | Anti-foaming agent | Present | 3.2 | A |
| Example 7 | 0.6 (0.19~2.42) | 19.05 (9.01~232.08) | Thermal fusion | Absent | Anti-foaming agent | Present | 5 | B |
| Example 8 | 0.6 (0.19~2.42) | 19.05 (9.01~232.08) | Thermal fusion | Absent | Anti-foaming agent | Present | 4.4 | A |
| Example 9 | 0.6 (0.19~2.42) | 19.05 (9.01~232.08) | Thermal fusion | Absent | Anti-foaming agent | Absent | 4.4 | A |
| Example 10 | 0.6 (0.19~2.42) | 19.05 (9.01~232.08) | Silicone | Present | Anti-foaming agent | Absent | 4.3 | A |
| Example 11 | 0.6 (0.19~2.42) | 19.05 (9.01~232.08) | Silicone | Absent | Anti-foaming agent | Absent | 4.4 | A |
| Example 12 | 0.6 (0.19~0.60) | 9.53 (9.01~232.08) | Thermal fusion | Absent | Anti-foaming agent | Present | 5 | B |
| Example 13 | 0.6 (0.19~0.91) | 19.05 (23.83~232.08) | Thermal fusion | Absent | Anti-foaming agent | Present | >5 | c |
| Example 14 | 1.5 (0.19~2.42) | 19.05 (22.53~232.08) | Thermal fusion | Absent | Anti-foaming agent | Present | >5 | c |

(continued)

| Example/ Comparative Example | Tube length | Tube thickness | Tube material | Tube branch | Anti-foaming function | Waste liquid line | Maximum internal pressure | Result |
|---|---|---|---|---|---|---|---|---|
| | m | mm | | | | | psi | |
| Example 15 | 0.6 (0.19~2.42) | 19.05 (9.01~232.08) | Thermal fusion | Absent | Anti-foaming agent | Present | 4.5 | A |
| Example 16 | 0.6 (0.19~18.73) | 19.05 (1.16~232.08) | Thermal fusion | Absent | Anti-foaming agent | Present | 1.5 | A |
| Comparative Example 1 | | | | | Anti-foaming agent | Present | | D |

In Example 1, the internal pressure in the culture tank could be maintained at less than 5 psi. The antibody productivity was also high.

In Example 2, although the internal pressure in the culture tank instantaneously reached 5 psi, it could be continued. The antibody productivity was also high.

In Example 3, the internal pressure in the culture tank could be maintained at less than 5 psi. The antibody productivity was also high.

In Example 4, the internal pressure in the culture tank could be maintained at less than 5 psi. Although the antibody productivity is inferior to that in the perfusion culture, the antibody could be produced.

In Examples 5 and 6, the internal pressure in the culture tank could be maintained at less than 5 psi.

In Example 7, although the internal pressure in the culture tank instantaneously reached 5 psi, it could be continued.

In Examples 8 to 11, the internal pressure in the culture tank could be maintained at less than 5 psi.

In Example 12, although the internal pressure in the culture tank instantaneously reached 5 psi, it could be continued.

In Examples 13 and 14, the internal pressure in the culture tank exceeded 5 psi, and the culture control was stopped. However, it was immediately below 5 psi, and the control was restarted, and as a result, the culture could be continued.

In Example 15, the bubble reached the exhaust filter attached to the culture tank, and the exhaust filter of the culture tank was clogged. However, the exhaust filter on the bubble trap container side continued to function, and thus the internal pressure in the culture tank could be maintained at less than 5 psi.

In Example 16, the bubble reached the exhaust filter attached to the culture tank, and the exhaust filter of the culture tank was clogged. However, the exhaust filter on the bubble trap container side continued to function, and thus the internal pressure in the culture tank could be maintained at less than 5 psi.

In Comparative Example 1, the bubble trap was immediately filled with bubbles since the volume thereof was small due to the presence of the bubble trap in the inside, the bubble reached the exhaust filter, the exit for the exhaust gas disappeared, and thus the culture could not be continued.

Explanation of References

1: culture container
2: bubble trap container
3: exhaust filter
4: tube
5: waste liquid line
6: waste liquid pump
7: waste liquid tank
8: vessel
9: macrosparger
10: microsparger
11: perfusion device
12: pressure sensor
13: upper surface aeration
14: supply culture medium
15: permeated liquid
16: anti-foaming agent
17: physical anti-foaming mechanism
18: tube clamp
19: aseptic connector

**Claims**

1. A method for producing a product, comprising:

   culturing a cell using a cell culture device to produce a product from the cell,
   wherein the cell culture device includes a culture container accommodating a cell suspension containing cells

and at least one or more bubble trap containers capable of accumulating bubbles, the one or more bubble trap containers being connected to an outside of the culture container.

2. The method for producing a product according to claim 1,
wherein at least one or more exhaust filters are connected to the bubble trap container.

3. The method for producing a product according to claim 1 or 2,
wherein a waste liquid line is connected to the bubble trap container.

4. The method for producing a product according to claim 1 or 2,
wherein a discharge port for a gas which is aerated to the culture container is only a line that connects the culture container and the bubble trap container.

5. The method for producing a product according to claim 3,
wherein the waste liquid line connected to the bubble trap container is connected to a waste liquid tank maintaining an aseptic state.

6. The method for producing a product according to claim 5,
wherein the waste liquid tank is allowed to be switched aseptically.

7. The method for producing a product according to claim 1 or 2,

   wherein an anti-foaming agent is added into the bubble trap container, or
   a physical anti-foaming mechanism is provided in the bubble trap container.

8. The method for producing a product according to claim 1 or 2,
wherein the following expression is satisfied,

$$Mi^2 \times Ma^{0.5}/S/200 \leq Vt \leq 10\ W$$

   in the expression,

   Vt indicates a bubble trap container capacity (L),
   Mi indicates an aeration amount (L/min) from a microsparger having a pore diameter of 1 $\mu$m to 200 $\mu$m,
   Ma indicates an aeration amount (L/min) of a macrosparger having a pore diameter of 0.1 mm to 10 mm,
   S indicates a gas-liquid interface area ($m^2$) of a culture solution, and
   W indicates a culture solution amount (L) in the culture container.

9. The method for producing a product according to claim 1 or 2, wherein the following expression is satisfied,

$$L \leq [P^2/(Mi^{1.5} + Ma)] \times S^{0.5}$$

   in the expression,

   L indicates a length (m) of a tube that connects the bubble trap container and the culture container,
   S indicates a gas-liquid interface area ($m^2$) of a culture solution,
   P indicates a thickness (mm) of a tube that connects the bubble trap container and the culture container,
   Mi indicates an aeration amount (L/min) from a microsparger having a pore diameter of 1 $\mu$m to 200 $\mu$m, and
   Ma indicates an aeration amount (L/min) of a macrosparger having a pore diameter of 0.1 mm to 10 mm.

10. The method for producing a product according to claim 1 or 2, wherein the following expression is satisfied,

$$(Mi^{1.5} + Ma) \times L \times S^{0.5}/10 \leq P \leq 50 \times Vt^{(1/3)}$$

P indicates a thickness (mm) of a tube that connects the bubble trap container and the culture container,

S indicates a gas-liquid interface area ($m^2$) of a culture solution,
L indicates a length (m) of a tube that connects the bubble trap container and the culture container,
Mi indicates an aeration amount (L/min) from a microsparger having a pore diameter of 1 $\mu$m to 200 $\mu$m,
Ma indicates an aeration amount (L/min) of a macrosparger having a pore diameter of 0.1 mm to 10 mm, and
Vt indicates a bubble trap container capacity (L).

**11.** The method for producing a product according to claim 1 or 2,
wherein the following expression is satisfied,

$$W^{0.5}/1{,}000 \leq E \leq Vc/S/100$$

in the expression,

E indicates a surface area ($m^2$) of an exhaust filter connected to the bubble trap container,
W indicates a culture solution amount (L) in the culture container,
Vc indicates a culture container volume (L), and
S indicates a gas-liquid interface area ($m^2$) of a culture solution.

**12.** The method for producing a product according to claim 1 or 2, wherein the following expression is satisfied,

$$1{,}000 \times S^2 \times Mi^2 \times Ma^{0.5} \times L/P^2/(Vc - W) \leq F \leq W/10$$

in the expression,

F indicates an upper surface aeration amount (L/min) flowing into the culture container,
Vc indicates a culture container volume (L),
W indicates a culture solution amount (L) in the culture container,
Mi indicates an aeration amount (L/min) from a microsparger having a pore diameter of 1 $\mu$m to 200 $\mu$m,
Ma indicates an aeration amount (L/min) of a macrosparger having a pore diameter of 0.1 mm to 10 mm,
L indicates a length (m) of a tube that connects the bubble trap container and the culture container,
P indicates a thickness (mm) of a tube that connects the bubble trap container and the culture container, and
S indicates a gas-liquid interface area ($m^2$) of a culture solution.

**13.** The method for producing a product according to claim 1 or 2, wherein the culture container is a single-use culture tank, and/or
the bubble trap container is a single-use bag.

**14.** The method for producing a product according to claim 1 or 2,
wherein the bubble trap container is a container that is sealed except for a pipe connected to the outside.

**15.** The method for producing a product according to claim 1 or 2,
wherein a viable cell density in the culture solution is $10 \times 10^6$ to $300 \times 10^6$ cells/mL.

**16.** The method for producing a product according to claim 1 or 2,
wherein a culture solution amount of the culture container is 10 L or more.

**17.** The method for producing a product according to claim 1 or 2,
wherein a method of culturing the cell is perfusion culture.

**18.** The method for producing a product according to claim 1 or 2,
wherein the cell is a CHO cell.

**19.** The method for producing a product according to claim 1 or 2,
wherein the product produced by the cell is an antibody.

**20.** The method for producing a product according to claim 1 or 2,

wherein a culture period is 14 days or more.

21. A cell culture device comprising:

a culture container accommodating a cell suspension containing cells; and
at least one or more bubble trap containers capable of accumulating bubbles, the one or more bubble trap containers being connected to an outside of the culture container by a pipe.

22. The cell culture device according to claim 21,
wherein at least one or more exhaust filters are connected to the bubble trap container.

23. The cell culture device according to claim 21 or 22,
wherein a waste liquid line is connected to the bubble trap container.

24. The cell culture device according to claim 21 or 22,
wherein a discharge port for a gas which is aerated to the culture container is only a line that connects the culture container and the bubble trap container.

25. The cell culture device according to claim 23,
wherein the waste liquid line connected to the bubble trap container is connected to a waste liquid tank maintaining an aseptic state.

26. The cell culture device according to claim 25,
wherein the waste liquid tank is allowed to be switched aseptically.

27. The cell culture device according to claim 21 or 22,
wherein a physical anti-foaming mechanism is provided in the bubble trap container.

28. The cell culture device according to claim 21 or 22,
wherein the following expression is satisfied,

$$\mathrm{Mi}^2 \times \mathrm{Ma}^{0.5}/\mathrm{S}/200 \leq \mathrm{Vt} \leq 10\,\mathrm{W}$$

in the expression,

Vt indicates a bubble trap container capacity (L),
Mi indicates an aeration amount (L/min) from a microsparger having a pore diameter of 1 $\mu$m to 200 $\mu$m,
Ma indicates an aeration amount (L/min) of a macrosparger having a pore diameter of 0.1 mm to 10 mm,
S indicates a gas-liquid interface area ($m^2$) of a culture solution, and
W indicates a culture solution amount (L) in the culture container.

29. The cell culture device according to claim 21 or 22,
wherein the following expression is satisfied,

$$\mathrm{L} \leq \mathrm{P}^2/(\mathrm{Mi}^{1.5} + \mathrm{Ma}) \times \mathrm{S}^{0.5}$$

in the expression,

L indicates a length (m) of a tube that connects the bubble trap container and the culture container,
S indicates a gas-liquid interface area ($m^2$) of a culture solution,
P indicates a thickness (mm) of a tube that connects the bubble trap container and the culture container,
Mi indicates an aeration amount (L/min) from a microsparger having a pore diameter of 1 $\mu$m to 200 $\mu$m, and
Ma indicates an aeration amount (L/min) of a macrosparger having a pore diameter of 0.1 mm to 10 mm.

30. The cell culture device according to claim 21 or 22, wherein the following expression is satisfied,

$$(\text{Mi}^{1.5} + \text{Ma}) \times \text{L} \times \text{S}^{0.5}/10 \leq \text{P} \leq 50 \times \text{Vt}^{(1/3)}$$

P indicates a thickness (mm) of a tube that connects the bubble trap container and the culture container,
S indicates a gas-liquid interface area (m$^2$) of a culture solution,
L indicates a length (m) of a tube that connects the bubble trap container and the culture container,
Mi indicates an aeration amount (L/min) from a microsparger having a pore diameter of 1 μm to 200 μm,
Ma indicates an aeration amount (L/min) of a macrosparger having a pore diameter of 0.1 mm to 10 mm, and
Vt indicates a bubble trap container capacity (L).

**31.** The cell culture device according to claim 21 or 22,
wherein the following expression is satisfied,

$$\text{W}^{0.5}/1{,}000 \leq \text{E} \leq \text{Vc}/\text{S}/100$$

in the expression,

E indicates a surface area E (m$^2$) of an exhaust filter connected to the bubble trap container,
W indicates a culture solution amount (L) in the culture container,
Vc indicates a culture container volume (L), and
S indicates a gas-liquid interface area (m$^2$) of a culture solution.

**32.** The cell culture device according to claim 21 or 22,
wherein a volume of the culture container is 10 L or more.

**33.** The cell culture device according to claim 21 or 22,
wherein the culture container is a single-use culture tank, and/or
the bubble trap container is a single-use bag.

**34.** The cell culture device according to claim 21 or 22,
wherein the bubble trap container is a container that is sealed except for a pipe connected to the outside.

# FIG. 1

# FIG. 2

# FIG. 3

# FIG. 4

## INTERNATIONAL SEARCH REPORT

International application No.

**PCT/JP2022/046000**

### A. CLASSIFICATION OF SUBJECT MATTER

*C12M 3/00*(2006.01)i; *C12N 5/10*(2006.01)i; *C12N 1/00*(2006.01)i; *C12P 21/08*(2006.01)i; *C12M 1/00*(2006.01)i; *C12M 1/107*(2006.01)i; *C12M 1/21*(2006.01)i

FI: C12P21/08; C12M3/00 Z; C12M1/00 D; C12M1/21; C12M1/107; C12N1/00 E; C12N1/00 A; C12N5/10

According to International Patent Classification (IPC) or to both national classification and IPC

### B. FIELDS SEARCHED

Minimum documentation searched (classification system followed by classification symbols)

C12M3/00; C12N5/10; C12N1/00; C12P21/08; C12M1/00;

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 57-5688 A (TORAY INDUSTRIES, INC.) 12 January 1982 (1982-01-12) p. 1, left column, line 12 to p. 2, lower right column, line 4, fig. 1, 2 | 1-6, 8-26, 28-34 |
| Y | p. 1, left column, line 12 to p. 2, lower right column, line 4, fig. 1, 2 | 7, 27 |
| Y | Microfilm of the specification and drawings annexed to the request of Japanese Utility Model Application No. 059702/1978 (Laid-open No. 1097/1979) (SHINKO INDUSTRIES CO., LTD.) 06 January 1979 (1979-01-06), p. 3, line 15 to p. 8, line 5, fig. 1, 2 | 7, 27 |
| A | entire text | 1-6, 8-26, 28-34 |
| Y | CN 206688452 U (UNIVERSITY TIANJIN SCIENCE & TECH.) 01 December 2017 (2017-12-01) paragraph [0015], fig. 1 | 7, 27 |
| A | entire text | 1-6, 8-26, 28-34 |
| Y | JP 2017-127226 A (TAISEI CORP.) 27 July 2017 (2017-07-27) paragraphs [0043]-[0046], fig. 2 | 7, 27 |
| A | entire text | 1-6, 8-26, 28-34 |

☑ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 February 2023** | **28 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| International application No. |
| --- |
| **PCT/JP2022/046000** |

### C.    DOCUMENTS CONSIDERED TO BE RELEVANT

| Category* | Citation of document, with indication, where appropriate, of the relevant passages | Relevant to claim No. |
| --- | --- | --- |
| A | JP 1-281072 A (HITACHI, LTD.) 13 November 1989 (1989-11-13)<br>entire text | 1-34 |
| A | JP 5-146286 A (AJINOMOTO CO., INC.) 15 June 1993 (1993-06-15)<br>entire text | 1-34 |
| A | JP 2012-170364 A (HITACHI PLANT TECHNOLOGIES LTD.) 10 September 2012 (2012-09-10)<br>entire text | 1-34 |
| A | JP 2018-19615 A (HITACHI, LTD.) 08 February 2018 (2018-02-08)<br>entire text | 1-34 |

Form PCT/ISA/210 (second sheet) (January 2015)

**INTERNATIONAL SEARCH REPORT**
Information on patent family members

International application No.

**PCT/JP2022/046000**

| Patent document cited in search report | | | Publication date (day/month/year) | Patent family member(s) | | | Publication date (day/month/year) |
|---|---|---|---|---|---|---|---|
| JP | 57-5688 | A | 12 January 1982 | (Family: none) | | | |
| JP | 54-1097 | U1 | 06 January 1979 | (Family: none) | | | |
| CN | 206688452 | U | 01 December 2017 | (Family: none) | | | |
| JP | 2017-127226 | A | 27 July 2017 | (Family: none) | | | |
| JP | 1-281072 | A | 13 November 1989 | US entire document WO EP KR | 5162204 1989/002458 336966 10-1990-0018366 | A A1 A1 A | |
| JP | 5-146286 | A | 15 June 1993 | US entire document | 5476573 | A | |
| JP | 2012-170364 | A | 10 September 2012 | (Family: none) | | | |
| JP | 2018-19615 | A | 08 February 2018 | WO entire document | 2018/025686 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016517691 A

- JP 2018019615 A